# EUROPEAN PATENT SPECIFICATION

(11) **EP 2 139 546 B1**
(45) Date of publication and mention of the grant of the patent: **14.11.2018**
(21) Application number: 08737011.0
(22) Date of filing: 17.04.2008
(51) Int. Cl.: A61M 11/00, A61M 16/14, A61M 16/00

(54) **VENTILATOR AEROSOL DELIVERY**
AEROSOLAUSGABE DURCH EINEN VENTILATOR
ADMINISTRATION D'AÉROSOL PAR VENTILATEUR

(30) Priority: 19.04.2007 US 925308 P; 10.04.2008 US 100487
(43) Date of publication of application: 06.01.2010
(73) Proprietor: Respironics Respiratory Drug Delivery (UK) Ltd, Bognor Regis West Sussex PO22 9SL (GB)
(72) Inventor: DENYER, Jonathan, S., H., West Sussex PO19 3PW (GB); PRINCE, Ivan, R., West Sussex PO19 3HB (GB)
(74) Representative: van Velzen, Maaike Mathilde
(86) International application number: PCT/GB2008/001350
(87) International publication number: WO 2008/129251

(56) References cited:
- DE-A1- 3 537 507
- US-A- 5 277 175
- US-A1- 2005 056 283
- US-B2- 7 188 621

## Description

### CROSS REFERENCE TO RELATED APPLICATIONS

Under the provisions of 35 U.S.C. § 119(e), this application claims the benefit of U.S. provisional patent application serial no. 60/925,308 filed April 19, 2007 and under the provisions of 35 U.S.C. § 120/365, this application claims the benefit of U.S. Application Serial No. 12/100,487, filed April 10, 2008.

### BACKGROUND OF THE INVENTION

### 1. Field of the Invention

The present invention pertains generally to administering an aerosolized medicament to a patient, and more particularly, to optimizing the delivery of a respiratory maneuver to increase the effectiveness of medicament delivery.

### 2. Description of the Related Art

It is known to deliver a medicament via a patient's respiratory tract. Such a delivery approach has allowed the development of therapeutic regimens for treating localized and systemic effects of diseases, especially diseases affecting the pulmonary system.

A nebulizer is a common device employed to deliver medicaments via the respiratory tract. Generally, a nebulizer converts an aqueous-based medicament into an aerosol which is inhaled by a patient. Nebulizers may employ different mechanisms for aerosolizing a medicament. For example, pneumatic (or jet) nebulizers employ compressed air to disperse the aqueous-based medicament into aerosol form. An ultrasonic nebulizer, in contrast, employs an electrical source to excite a piezoelectric element which generates high frequency vibrations. These vibrations cause small droplets to separate from the surface of the aqueous-based medicament, thus forming an aerosol. Vibrating mesh technology (VMT) nebulizers employ a mesh having a number of microscopic apertures therein. A vibration is induced in the mesh (or in another component within the nebulizer) which causes the aqueous-based medicament to be extruded through the mesh apertures. The aqueous-based medicament is ejected from the mesh apertures in aerosol form.

It is important that the dosage of medicament prescribed by a physician be administered in a reproducible and precise manner. Reproducibility and precision are greatly dependent upon one or more respiration maneuvers performed during administration of the medicament. The terms "respiration maneuver", "maneuver", and derivatives thereof refer to a number of respiratory cycle characteristics and/or dosing characteristics related to the administration of a partial and/or complete dose of a medicament. The term respiration maneuver may be understood to encompass, for example and without limitation, the number of breathes per minute (BPM), the inspiratory flow rate, the tidal volume, the volume of aerosol delivered to the patient, the aerosol pulse duration, and the duration of the dosing event.

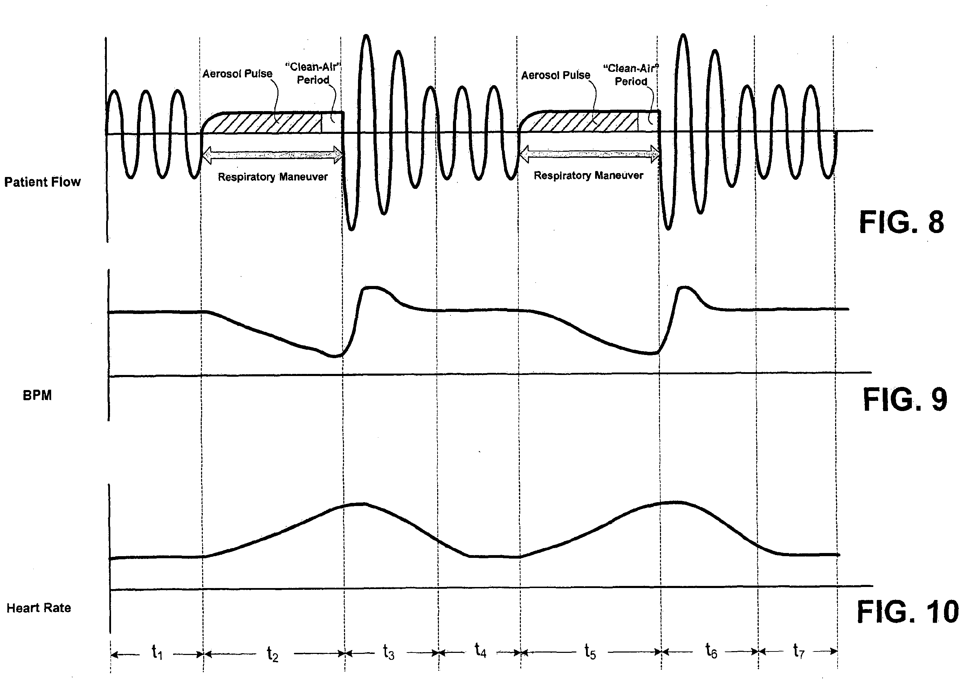

U.S. Patent Application Number 10/535,597 to Denyer et al., teaches a respiration maneuver which improves the precision and repeatability of medicament delivery to a patient. Generally, Denyer et al. teach a method of generating signals in a drug delivery apparatus through which a patient is receiving a dose of medicament. These signals may be used as feedback to aid the patient in completing a desired respiration maneuver. Denyer et al., however, is generally directed to patients capable of spontaneous breathing (e.g., non-ventilated patients). Mechanically ventilated patients, in contrast, may not be able to adequately respond to the generated feedback signals, and thus fail to complete the desired respiratory maneuver. Further, US 7,188,621 B2 describes a low noise portable ventilator. The portable ventilator can be used by patients requiring continuous breathing assistance to improve their mobility. The purpose of US 7,188,621 B2 is to reduce noise. Further, DE 35 37 507 discloses a nebulizer and ventilator assembly. It concerns a device for the inhalation of medicaments with assisted intermittent positive pressure ventilation for the treatment of patients with reduced or disturbed pulmonary function. The purpose of DE 35 37 507 is to provide an inhalation ventilator for aerosol therapy of patients which can automatically be adjusted. Further, US 5,277,175 describes a nebulizer/ventilator assembly in which the nebulizer is turned on and off in one of four modes depicted in Figure 10. Mode 1 is continuously on; mode 2 is synchronized with the breathing pattern, mode 3 is inverse the breathing pattern; and mode 4 is offset relative to the breathing pattern. Further, US2005/0056283A1 describes a method for integrating a ventilator device with a monitoring device and other medical devices that allows the function of the monitoring device and medical devices to automatically change based on initiation of a respiratory therapy procedure.

The delivery of an aerosolized medicament to a patient through a ventilator circuit can be inefficient. In some cases, only ten to thirty percent of the nominal dose actually reaches the patient. The remaining medicament may be, for instance, carried away in the exhalation stream or lost to impaction with the ventilator circuit, endotracheal tube, and the patient's upper airway.

Attempts made to reduce medicament lost in the exhalation stream include, for example, triggering the aerosol generator only during inhalation, increasing the sedimentation time, and orienting the patient's position during administration of the aerosolized medicament. Attempts made to reduce medicament loss due to impaction include, for example, relocating the aerosol generator (e.g., a nebulizer) between the ventilator circuit and patient interface device (e.g., endotracheal tube; mask; etc.), suspending humidification during aerosol generation, and reducing the inhalation flow rate. Each of these attempts produce less than desirable results and generally requires an affirmative act by the clinician. For example, a clinician must manually adjust the ventilator's settings to reduce the inhalation flow rate.

Accordingly, a need exists for an apparatus and method for providing improved delivery of an aerosolized medicament to a ventilated patient which overcomes these and other problems associated with known systems.

### SUMMARY OF THE INVENTION

In accordance with an example which does not form part of the invention claimed, a method for delivering a medicament to a mechanically ventilated patient receiving a flow of breathing gas comprises delivering a respiratory maneuver to such a patient, monitoring the physiological effects of the respiratory maneuver on such patient, automatically establishing an optimized respiratory maneuver in response to the monitored physiological effects, and delivering the optimized respiratory maneuver to such a patient.

According to another example which does not form part of the invention claimed, a method for coordinating delivery of a flow of gas and delivery of a medicament to a mechanically ventilated patient comprises generating the flow of gas with a flow generator, determining a number of first operational parameters associated with the flow generator, establishing a respiratory maneuver, wherein the respiratory maneuver includes a number of second operational parameters associated with the flow generator and a number of operational parameters associated with a medicament delivery device, delivering the respiratory maneuver to such patient, wherein the number of second operational parameters associated with the flow generator and the number of operational parameters associated with the medicament delivery device are implemented in a coordinated fashion, monitoring such patient's physiological reaction to the respiratory maneuver, and automatically establishing an optimized respiratory maneuver responsive to such patient's physiological reaction.

According to the present invention, a system for delivering an aerosolized medicament to a ventilated patient comprises a ventilator structured to produce a flow of breathing gas, a nebulizer structured to aerosolize a medicament, a monitoring device structured to generate an output signal associated with a response of such a patient to receipt of the aerosolized medicament, and a controller operatively coupled with the ventilator, nebulizer, and monitoring device, wherein the controller is adapted to coordinate operation of the ventilator and the nebulizer to produce a respiratory maneuver, and responsive to the output signal from the monitoring device, automatically establish ventilator settings and nebulizer settings to optimize delivery of the aerosolized medicament to such patient. The system further comprises a number of sensors arranged to be in communication with the monitoring device, the sensors being arranged to be applied to the patient to track the patient's physiological state.

### BRIEF DESCRIPTION OF THE DRAWINGS

FIG. 1 is a schematic view of a system adapted to provide a regimen of respiratory therapy according to one embodiment of the present invention.
FIG. 2 illustrates an operational process for delivering a medicament to a mechanically ventilated patient according to one embodiment of the present invention.
FIG. 3 illustrates an operational process for delivering a medicament to a mechanically ventilated patient according to another embodiment of the present invention.
FIG. 4 is a graph illustrating delivery of an aerosolized medicament to a mechanically ventilated patient according to one embodiment of the present invention.
FIG. 5 is a graph illustrating lung compliance related to delivery of the aerosolized medicament of FIG. 4.
FIG. 6 is a graph illustrating lung resistance related to delivery of the aerosolized medicament of FIG. 4.
FIG. 7 is a graph illustrating circuit pressure related to delivery of the aerosolized medicament of FIG. 4.
FIG. 8 is a graph illustrating an aerosolized medicament treatment to a mechanically ventilated patient according to one embodiment of the present invention.
FIG. 9 is graph illustrating breathes per minute related to the aerosolized medicament treatment of FIG. 8.
FIG. 10 is graph illustrating heart rate related to the aerosolized medicament treatment of FIG. 8.

### DETAILED DESCRIPTION OF THE EXEMPLARY EMBODIMENTS

Directional phrases used herein, such as, for example, left, right, clockwise, counterclockwise, top, bottom, up, down, and derivatives thereof, relate to the orientation of the elements shown in the drawings and are not limiting upon the claims unless expressly recited therein.

As employed herein, the term "number" shall mean one or more than one and the singular form of "a", "an", and "the" include plural referents unless the context clearly indicates otherwise.

As employed herein, the statement that two or more parts are "connected" or "coupled" together shall mean that the parts are joined together either directly or joined together through one or more intermediate parts. Further, as employed herein, the statement that two or more parts are "attached" shall mean that the parts are joined together directly.

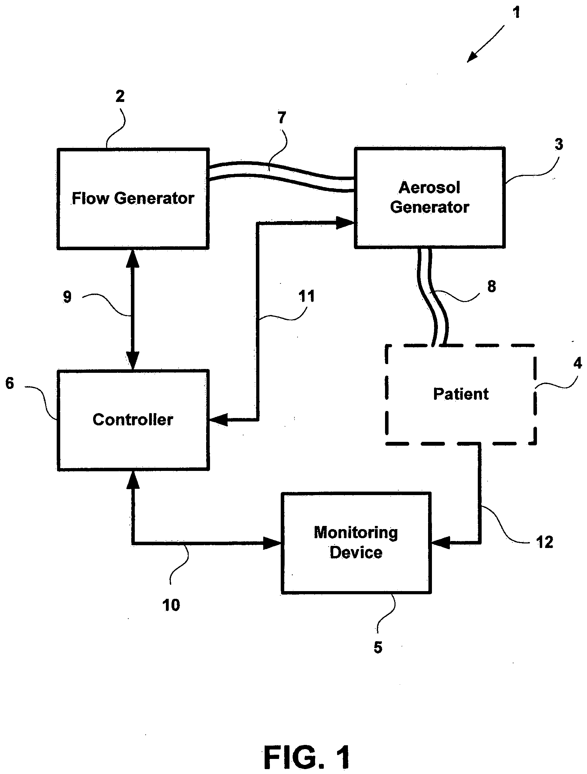

A system 1 for delivering an aerosolized medicament to a ventilated patient according to one embodiment is shown in FIG. 1. System 1 includes a flow generator 2, an aerosol generator 3, a patient monitoring device 5, and a controller 6.

Flow generator 2 is structured to generate a flow of breathing gas which is communicated through a patient circuit 7 to a patient 4. In the current embodiment, a ventilator 2, such as a BiPap® Vision® ventilator or a BiPap® Focus® ventilator, each available from Respironics, Inc. of Murrysville, Pennsylvania, is employed as the flow generator, although it is contemplated that other devices may be used. As seen in FIG. 1, the flow of breathing gas is delivered to patient 4 via a patient interface device 8, such as an endotracheal tube 8, which is in fluid communication with patient circuit 7. It is contemplated that other patient interfaces may be employed. Aerosol generator 3 is located downstream of patient circuit 7; here between patient circuit 7 and endotracheal tube 8. As a result, impaction of aerosolized medicament within patient circuit 7 is eliminated. A nebulizer 3, such as a Sidestream® nebulizer available from Respironics, Inc., is employed as the aerosol generator in the current embodiment, although it is contemplated that other devices may be used.

Monitoring device 5 is structured to track a patient's 4 physiological state, for example, in response to a delivered respiratory maneuver. Examples of such a monitoring device 5 includes a NICO® Cardiopulmonary Management System and a Capnogard® Capnograph, each available from Respironics, Inc., although it is contemplated that other devices may be used. A number of sensors (not shown in FIG. 1) are typically applied to patient 4 and communicate with monitoring device 5 via signal line 12. Although illustrated as communicating via a hard-wired connection, it is contemplated that monitoring device 5 may wirelessly communicate with the number of sensors while remaining within the scope of the present invention. Monitoring device 5 may receive signals associated with, for example and without limitation, lung compliance, airway resistance, circuit pressure, respiratory rate, minute volume, partial pressure of oxygen in arterial blood, partial pressure of carbon dioxide in arterial blood, and percentage of oxygen saturation in arterial blood.

Controller 6 may include a processor, a memory, and a user interface device (not shown), among others. Generally, the processor is adapted to implement a number of routines for optimizing the delivery of a respiratory maneuver. The routines may be in any of a variety of forms such as, without limitation, software, firmware, and the like which are executable by the processor. For example, the processor may be adapted to implement a routine for coordinating the operations of ventilator 2 and nebulizer 3. The memory can be any of a variety of types of internal and/or external storage media such as, without limitation, RAM, ROM, EPROM(s), EEPROMS(s), and the like that provide a register for data storage such as in the fashion of an internal storage area of a computer, and can be volatile memory or nonvolatile memory. The memory provides storage for, among others, the number of routines executable by the processor. The user interface may include, without limitation, a mouse, a keyboard, and/or a display.

Controller 6 is structured to communicate with ventilator 2, nebulizer 3, and monitoring device 5. In the current embodiment, controller 6 communicates with ventilator 2 via signal line 9, with nebulizer 3 via signal line 11, and with monitoring device 5 via signal line 10. Although illustrated as communicating via hard-wired connections, it is contemplated that system 1 may incorporate one or more wireless communication devices (e.g., controller 6 may wirelessly communicate ventilator 2, nebulizer 3, and/or monitoring device 5), while remaining within the scope of the present invention.

Although shown as discrete components in FIG. 1, it is contemplated that one or more of the devices and/or their functions may be combined into a single device while remaining within the scope of the present invention. For example, the functions of controller 6 may be incorporated into and carried out by ventilator 2.

System 1 is adapted such that the operation of ventilator 2 and nebulizer 3 are synchronized. As will be discussed in more detail herein, controller 6 is adapted, without limitation, to determine the breathing cycle of patient 4, activate nebulizer 3 during a portion of the patient's breathing cycle, and control the operation of the ventilator 2 during aerosol delivery. Controller 6 is also adapted to optimize the operations of ventilator 2 and nebulizer 3 in response to a patient's physiological reaction to the respiratory maneuver and/or to the delivered medicament.

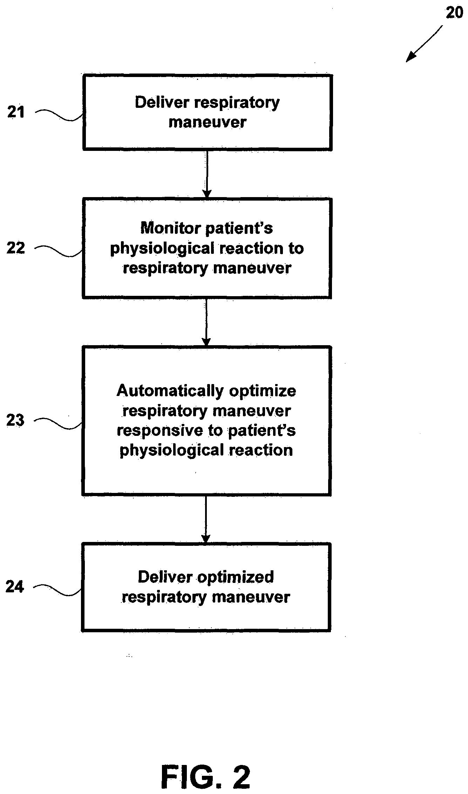

FIG. 2 illustrates an operational process for delivering a medicament to a mechanically ventilated patient according to one embodiment of the present invention. For example and without limitation, the patient in the current embodiment is a 154 pound (70 kg) average adult-sized male patient with a total lung capacity (TLC) of 6 liters (L), a vital capacity (VC) of 4.8L, and a residual volume (RV) of 1.2L. During normal breathing, it is assumed that such a patient has a tidal volume of five hundred milliliters (500 mL), a respiratory rate of ten breaths per minute (10 BPM), and a minute volume of five liters per minute (5 L/min). It should be apparent to one skilled in the art that the invention is in no way limited to such a patient and may easily be adapted for any patient.

At operation 21, a respiratory maneuver is delivered to such patient. In the current embodiment, controller 6 is adapted to synchronize the operation of the ventilator 2 and the nebulizer 3 such that a desired respiratory maneuver is produced. For example, controller 6 adjusts ventilator 2 to deliver a flow of breathing gas at a rate of twenty-four liters per minute (24 L/min) for a time period of approximately three seconds (3 sec). This maneuver provides a tidal volume of 1.2L, which is approximately 25% of the patient's vital capacity. Additionally, controller 6 activates nebulizer 3 during the initial stages of the inhalation phase causing the medicament to be aerosolize and deactivates nebulizer 3 prior to the end of the inhalation phase (e.g., two seconds prior to the end of the inhalation stage) allowing a period of "clean air" to be delivered after the aerosol pulse.

At operation 22, the patient's physiological reaction to the respiratory maneuver and the delivered medicament is monitored. In the current embodiment, monitoring device 5 tracks the patient's physiological reaction to the respiratory maneuver and to the delivered medicament. A number of sensors (not shown in FIG. 1) are applied to the patient 4 and communicate with monitoring device 5 via signal line 12. Monitoring device 5 may receive signals associated with, for example and without limitation, lung compliance, airway resistance, circuit pressure, respiratory rate, minute volume, partial pressure of oxygen in arterial blood, partial pressure of carbon dioxide in arterial blood, and percentage of oxygen saturation in arterial blood.

At operation 23, the respiratory maneuver is automatically optimized in response to the patient's physiological reaction monitored in operation 22. In the current embodiment, monitoring device 5 communicates a number of signals associated with the patient's physiological reaction to controller 6 via signal line 10. Controller 6 employs these signals to calculate an optimized respiratory maneuver. For example, controller 6 may use signals associated with lung compliance, airway resistance, and circuit pressure to calculate a new inhaled volume that will be delivered by the optimized respiratory maneuver.

At operation 24, the optimized respiratory maneuver is delivered to the patient. In the current embodiment, controller 6 adjusts the settings of ventilator 2 and/or nebulizer 3 to deliver optimized respiratory maneuver. Controller 6 may adjust ventilator 2, for instance, to deliver a tidal volume of approximately 80% of the patient's vital capacity. For example, ventilator 2 is adjusted to deliver a flow rate of approximately fifty liters per minute (50 L/min) for a time period of approximately four-and-one-half seconds (4.5 sec.) and to activate nebulizer 3 during the initial stages of the inhalation phase and deactivate nebulizer 3 prior to the end of the inhalation phase (thus allowing a period of "clean air" to be delivered after the aerosol pulse).

It is contemplated that operations 22 - 24 may be repeated as many times necessary during the course of a treatment. For instance, multiple respiratory maneuvers may be required to deliver the entire prescribed dose of medicament during a single treatment (i.e., a treatment may consist of any number of respiratory maneuvers). Additionally, it is contemplated that the optimized maneuver settings associated with the last respiratory maneuver of a treatment may be used for the first respiratory maneuver of a subsequent treatment. As a result, aerosol delivery of the subsequent treatment is optimized (e.g., system 1 automatically "learns" patient's condition).

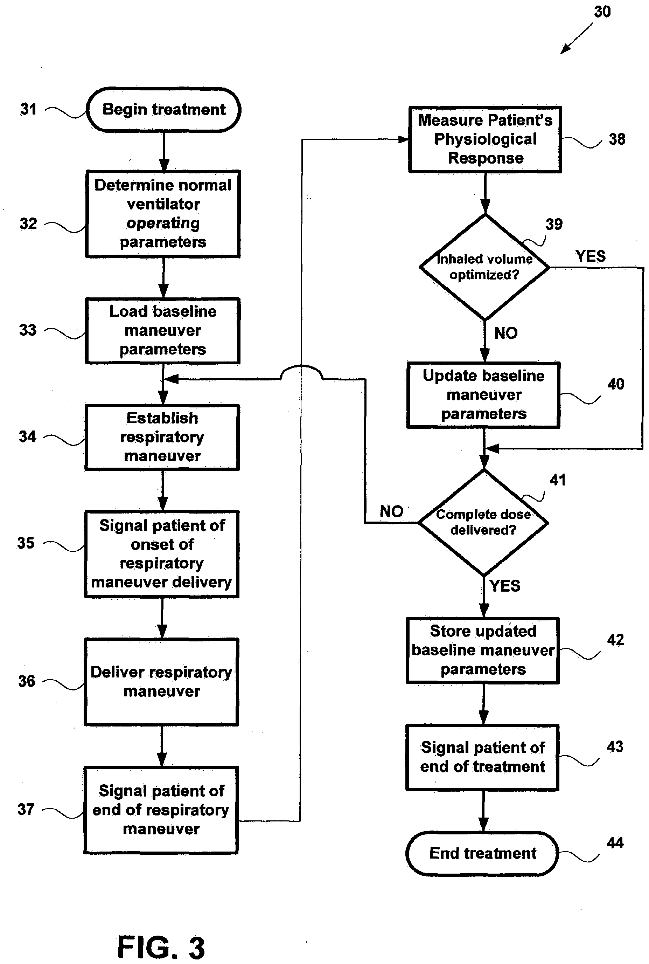

FIG. 3 illustrates an operational process 30 for delivering an aerosolized medicament according to another embodiment of the present invention. At operation 31 the treatment begins when a clinician loads a preset dose of aqueous-base medicament into the delivery system and designates the time period over which the medicament is to be delivered. In the current embodiment, for example, a clinician loads a preset dose of albuterol into nebulizer 3 and indicates that the dose is to be delivered over a period of 5 to 30 minutes.

Operational control then passes to operation 32 where the normal parameters of the ventilator are determined. In the current embodiment, controller 6 receives the normal parameters (e.g., the parameters associated with of a steady-state breathing cycle) from ventilator 2 via signal line 9. In the current embodiment, example, ventilator 2 is set to deliver a tidal volume of 500 mL at a flow rate of 25 L/min with an inhalation time of 1.2 seconds.

After receiving the normal parameters, operational control passes to operation 33 where the baseline respiratory maneuver parameters are loaded. In the current embodiment, the baseline respiratory maneuver parameters are derived from the normal ventilator settings and are loaded into controller 6. For example and without limitation, the tidal volume parameter for the baseline respiratory maneuver is 1.5 times the normal tidal volume parameter, the inhalation flow rate parameter for the baseline respiratory maneuver is 50% of the normal inhalation flow rate parameter, and an aerosol pulse time parameter for the baseline respiratory maneuver is approximately 25% of the normal inhalation time parameter and includes a maximum two-second "clean air" period.

After the baseline maneuver parameters are loaded, the respiratory maneuver is established in operation 34. In the current embodiment, controller 6 establishes the respiratory maneuver at this stage using the baseline maneuver parameters loaded in operation 33 and the normal operating parameters from operation 32. Continuing the above example, the tidal volume for the baseline respiratory maneuver is determined by the controller to be 750 mL (i.e., 1.5 x 500 mL), the inhalation flow rate is determined to be 12.5 L/min (i.e., 50% of 25 L/min), the inhalation time is determined to be 3.6 seconds, and a aerosol pulse time is determined to be 0.9 seconds (i.e., approximately 25% of 3.6 seconds.).

After the respiratory maneuver is established, operational control passes to operation 35 where a signal is generated informing the patient that the onset of the respiratory maneuver is approaching. In the current embodiment, for instance, an audible signal is generated. It is contemplated that any signal (such as without limitation, an audible signal, visual signal, and/or a tactile signal) or combination of signals may be used to inform the patient that the onset of the respiratory maneuver is approaching while remaining within the scope of the present invention.

After signal is generated in operation 35, operational control passes to operation 36 where the respiratory maneuver is delivered to the patient. In the current embodiment, controller 6 is adapted to deliver the respiratory maneuver established in operation 34. Accordingly, controller 6 sends a trigger signal and a flow time signal to ventilator 2 via signal line 9. In response, ventilator 2 reduces the inhalation flow rate from 25 L/min to 12.5 L/min and increases the tidal volume from 500 mL to 750 mL. Additionally, controller 6 sends an aerosol trigger signal to nebulizer 3 via signal line 11. In response, nebulizer 3 aerosolizes the aqueous-base medicament for 0.9 seconds (i.e., the aerosol pulse time). The aerosolized medicament is transported by the flow of breathing gas generated by ventilator 2 to the patient's lungs via endotracheal tube 8 and the patient's upper airway. Nebulizer 3 is deactivated prior to the end of the inhalation phase to provide a "clean air" period.

It is contemplated that controller 6 may issue other commands prior to, or subsequent to, delivery of the respiratory maneuver. Controller 6, for example, may issue a first command suspending the ventilator's humidifier operation prior to delivery of the respiratory maneuver and a second command reinstating humidifier operation subsequent to delivery of the respiratory maneuver.

After the respiratory maneuver is delivered, operational control passes to operation 37 where a signal is generated to inform the patient that the current respiratory maneuver has ended. In the current embodiment, for instance, an audible signal is generated. It is contemplated that any signal (such as without limitation, an audible signal, visual signal, and/or a tactile signal) or combination of signals may be used to inform the patient that the respiratory maneuver has ended while remaining within the scope of the present invention.

Next, operational control passes to operation 38 where the patient's response to the respiratory maneuver, and to the delivered medicament, is measured. In the current embodiment, ventilator 2 is employed to monitor several parameters such as, without limitation, lung compliance, airway resistance, and circuit pressure. These parameters are communicated to controller 6 via signal line 9. Additionally, monitoring device 5 is adapted to monitor several parameters such as, without limitation, breathes per minute (BPM), minute volume, saturation of oxygen (S_{A}O₂), carbon dioxide (CO₂), and heart rate. These parameters are communicated from monitoring device 5 to controller 6 via signal line 10.

After the patient's response is measured, a determination is made at operation 39 as to whether the inhaled volume is optimized. In the current embodiment, a tidal volume that is 80% of the patient's vital capacity is selected as a target and lung compliance, airway resistance, and circuit pressure parameters, among others, are used to determine if the inhalation volume for the next maneuver should be increased/decreased. Additionally, the BPM, minute volume, S_{A}O₂, CO₂, and heart rate parameters are used to determine whether adjustments can be made to the parameters of the next respiratory maneuver to minimize patient stress. It is contemplated that a metric other than or in addition to the tidal volume that is 80% of the patient's vital capacity may be employed to determine whether the respiratory maneuver is optimized.

If the inhaled volume is not optimized (e.g., in the current example, if the tidal volume setting of the respiratory maneuver is not 80% of the patient's vital capacity), operation 39 branches NO and operational control passes to operation 40 where the baseline maneuver parameters are updated. In the current embodiment, one or more of the baseline maneuver parameters are replaced by the respiratory parameters established in operation 34. After the baseline maneuver parameters are updated at operation 40, or if operation 39 branches YES, operational control passes to operation 41.

At operation 41, a determination is made as to whether the complete dose of aqueous-base medicament which was loaded into the delivery system by the clinician at operation 31 has been delivered. If a portion of the medicament remains, operation 41 branches NO and operational control returns to operation 34. At operation 34, the respiratory maneuver is established. It should be noted that this respiratory maneuver will include the updated baseline parameters, if any, from operation 40. Operations 34 through 41 are repeated until the complete dose of medicament is delivered to the patient and/or the time period over which the medicament is to be delivered (as designated in operation 31) expires. In the current embodiment, the time period between deliveries of respiratory maneuvers depends upon the patient's response to the previous respiratory maneuver. For instance, the first respiratory maneuver may have caused the patient's heart rate to increase. A subsequent respiratory maneuver will not be delivered until the patient's heart rate returns to an acceptable level.

If it is determined at operation 41 that the complete dose of aqueous-based medicament that was loaded into the nebulizer 3 has been delivered, operation 41 branches YES and operational control is passed to operation 42. At operation 42 the latest set of updated baseline parameters (as determined at operation 40), are stored. Accordingly, medicament delivery for subsequent treatments (e.g., the next time operational process 30 is implemented) will be optimized.

After the updated default settings are stored, operational control passes to operation 43 where a signal is generated indicating that current treatment has been completed. In the current embodiment, for instance, an audible signal is generated. It is contemplated that any signal (such as without limitation, an audible signal, visual signal, and/or a tactile signal) or combination of signals may be used to indicate that the current treatment has been completed while remaining within the scope of the present invention. It is preferable (though not necessary) that the signals generated in operation 35 (the signal informing the patient of the onset of the respiratory maneuver), operation 37 (the signal informing the patient of the end of the respiratory maneuver), and operation 43 (the signal informing the patient of the end of the current treatment) are sufficiently different so as not to confuse the patient as to the meaning of the generated signal.

After the signal indicating that the treatment has been completed is generated, operation 44 terminates operational process 30. In the current embodiment, controller 6 communicates to ventilator 2 that the treatment is completed. As a result, the parameters associated with of the patient's steady-state breathing cycle are loaded into ventilator 2. For instance, in the current example, ventilator 2 is reset to deliver a tidal volume of 500 mL at a flow rate of 25 liters per minute (L/min) at an inhalation time of 1.2 seconds. It is contemplated that these parameters may be updated as necessary to reflect any change in the patient's condition resulting from the treatment administration. For example, the effects of the administered medicament on the patient may necessitate that the tidal volume parameter be increase to 600 mL.

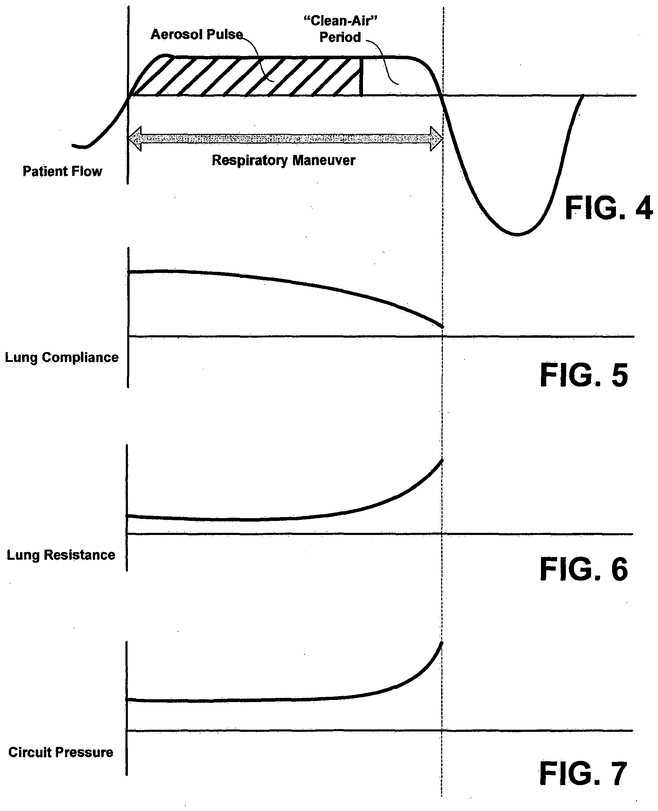

FIG. 4 is a graphical illustration of a single respiratory maneuver for the delivery of an aerosolized medicament to a mechanically ventilated patient according to one embodiment of the present invention. As seen in FIG. 4, the respiratory maneuver is applied during the inspiratory phase. The respiratory maneuver includes an aerosol pulse followed by a "clean-air" period, which immediately precedes the expiratory phase. FIGS. 5 - 7 illustrate lung compliance, lung resistance, and circuit pressure related to delivery of the aerosolized medicament shown in FIG. 4. Referring to FIG. 5, it can be seen that the amount of lung compliance generally decreases over the inhalation time period. In contrast, referring to FIGS. 6 and 7, it can be seen that lung resistance and circuit pressure remain relatively constant for the first portion of the inhalation phase, but then increase rapidly near the termination of the inhalation phase.

FIG. 8 illustrates the flow of breathing gas delivered to a mechanically ventilated patient during an aerosolized medicament treatment according to one embodiment of the present invention. FIGS. 9 and 10 illustrate breathes per minute BPM and heart rate, respectively, of such a patient in response to the aerosolized medicament treatment of FIG. 8.

During time period "t₁", a steady-state flow of breathing gas is delivered to a patient to maintain normal breathing. The patient's BPM and heart rate remain substantially constant.

During time period "t₂", a respiratory maneuver is delivered to the patient. As seen in FIG. 8, the respiratory maneuver includes a decrease in the normal (i.e., steady-state) flow rate, an increase in the normal (i.e., steady-state) tidal volume, and an aerosol pulse with a "clean-air" period. In response to the respiratory maneuver, the patient's BPM decreases and the patient's heart rate increases. The normal tidal volume and the normal flow rate associated with flow of gas are decreased for a first predetermined period and the nebulizer is activated for a second predetermined period which has a duration that is less than the duration of the first predetermined period. In the current embodiment, the duration of the second predetermined period (i.e., the duration of the aerosol pulse) is less than the duration of the first predetermined period (i.e., the duration of the entire respiratory maneuver) by the duration of the "clean-air" period.

After the respiratory maneuver is delivered, the amount of breathing gas delivered to the patient is somewhat transient. As seen in FIG. 8, a larger flow of breathing gas is delivered to the patient at the beginning of time period "t₃". In response, the patient's BPM increases and the patient's heart rate decreases. During time period "t₃", the amount of breathing gas delivered to the patient slowly settles until a steady-state is reached. In response, the patient's BPM and heart decrease return to a substantially constant level.

After the patient's BPM and heart rate have returned to a constant level (as shown at time period "t₄"), another respiratory maneuver is delivered during time period "t₅". In the current embodiment, this respiratory maneuver is optimized and may includes a decrease in the flow rate, an increase in the tidal volume, and an aerosol pulse with a "clean-air" period which may be different than that of the respiratory maneuver delivered during time period "t₂". In response to the respiratory maneuver delivered during time period "t₅", the patient's BPM decreases and the patient's heart rate increases.

After the respiratory maneuver is delivered, the amount of breathing gas delivered to the patient is somewhat transient. A larger flow of breathing gas is delivered to the patient at the beginning of time period "t₆". In response, the patient's BPM increases and the patient's heart rate decreases. During time period "t₆", the amount of breathing gas delivered to the patient slowly settles until a steady-state is reached. In response, the patient's BPM and heart decrease return to a substantially constant level (as shown during time period "t₇".

Although FIGS. 8 - 10 only illustrate the delivery of two respiratory maneuvers, it is contemplated that any number of respiratory maneuvers may be delivered to the patient while remaining within the scope of the present invention.

While specific respiratory maneuvers were employed to illustrate the disclosed embodiments, it should be apparent to one skilled in the art that the specific respiratory maneuver selected for administration to a patient may encompass a vast range of values. For example, it is contemplated that any number of respiratory maneuvers may be selected in which a tidal volume between 0 - 4000 milliliters, at a flow rate between 0 - 99 liters per minute, at an inhalation time between 0 - 10 seconds are delivered. Generally, it is contemplated that a selected respiratory maneuver will be selected in which a tidal volume between 100 - 1000 milliliters, at a flow rate between 10 - 50 liters per minute, at an inhalation time between 0.5 and 5 seconds is delivered.

## Claims

1. A system (1) for delivering an aerosolized medicament to a ventilated patient, comprising:
a ventilator (2) structured to produce a flow of breathing gas;
a nebulizer (3) structured to aerosolize a medicament;
a monitoring device (5) structured to generate an output signal; and
a controller (6) operatively coupled with the ventilator (2), nebulizer (3), and monitoring device (5), wherein the controller (6) is adapted to:
coordinate operation of the ventilator (2) and the nebulizer (3) to produce a respiratory maneuver;
**characterized in that**:
the system (1) further comprises a number of sensors arranged to be in communication with the monitoring device (5), the sensors being arranged to be applied to the patient (4) to track the patient's physiological state;
wherein the output signal is associated with a response of such a patient to receipt of the aerosolized medicament; and
wherein the controller (6) is further adapted to, responsive to the output signal from the monitoring device (5), automatically establish ventilator (2) settings and nebulizer (3) settings to optimize delivery of the aerosolized medicament to such patient.

2. The system (1) of claim 1, wherein the respiratory maneuver comprises:
(i) altering a normal tidal volume and a normal flow rate associated with flow of gas during a first predetermined period,
(ii) activating a drug delivery device structured to aerosolize the medicament during a second predetermined period, wherein the second predetermined period has a duration that is less than a duration of the first predetermined period, and
(iii) delivering the aerosolized medicament to such patient via the flow of gas.

3. The system (1) of claim 2, wherein altering the normal tidal volume comprises increasing the normal tidal volume.

4. The system (1) of claim 2, wherein altering the normal flow rate comprises decreasing the normal flow rate.

5. The system (1) of claim 2, wherein altering the normal tidal volume, altering the normal flow rate, and activating the drug delivery device occur substantially simultaneously.

6. The system (1) according to claim 1 wherein the monitoring device (5) is arranged to communicate wirelessly with the number of sensors.

7. The system (1) according to claim 1 wherein the monitoring device (5) is arranged to receive signals associated with at least one of lung compliance, airway resistance, circuit pressure, respiratory rate, minute volume, partial pressure of oxygen in arterial blood, partial pressure of carbon dioxide in arterial blood, and percentage of oxygen saturation in arterial blood.

8. The system (1) according to claim 1, wherein the system (1) is further arranged to generate an indication to a patient prior to the delivery of the respiratory maneuver and the optimized respiratory maneuver.

9. The system (1) according to claim 1, wherein the system (1) is further arranged to deactivate the nebulizer (3) prior to an end of an inhalation phase to provide a 'clean air' period.

10. The system (1) according to claim 1, wherein the system (1) is further arranged to generate a further indication to inform a patient that the respiratory maneuver has ended.

11. The system (1) according to claim 8 or 10, wherein the indication and/or further indication are an audible signal, a visual signal, and/or a tactile signal.

12. The system (1) according to claim 1, wherein the system (1) is arranged to adjust a time period between the delivery of the respiratory maneuver and a subsequent respiratory maneuver in dependence of the patient's response to the respiratory maneuver.

13. The system (1) according to claim 12 wherein the patient's response to the respiratory maneuver includes the patient's heart rate.

## Patentansprüche

1. System (1) zur Abgabe eines Medikament-Aerosols an einen beatmeten Patienten, umfassend:
ein Beatmungsgerät (2), das konstruiert ist, um eine Atemgasströmung zu erzeugen;
einen Vernebler (3), der konstruiert ist, um ein Medikament zu vernebeln;
eine Überwachungsvorrichtung (5), der konstruiert ist, um ein Ausgabesignal zu erzeugen; und
eine Steuereinheit (6), die betriebsfähig mit dem Beatmungsgerät (2), dem Vernebler (3) und der Überwachungsvorrichtung (5) gekoppelt ist, wobei die Steuereinheit (6) ausgelegt ist zum:
Koordinieren des Betriebs des Beatmungsgeräts (2) und des Verneblers (3), um ein Atmungsmanöver zu erzeugen;
**dadurch gekennzeichnet, dass**:
das System (1) ferner eine Anzahl von Sensoren umfasst, die angeordnet sind, um in Verbindung mit der Überwachungsvorrichtung (5) zu stehen, wobei die Sensoren angeordnet sind, um auf den Patienten (4) angewendet zu werden, um den physiologischen Zustand des Patienten zu verfolgen;
wobei das Ausgangssignal einer Reaktion eines derartigen Patienten auf den Empfang des Medikament-Aerosols zugeordnet ist; und
wobei die Steuereinheit (6) ferner dafür ausgelegt ist, als Reaktion auf das Ausgangssignal von der Überwachungsvorrichtung (5) automatisch Einstellungen des Beatmungsgeräts (2) und Einstellungen des Verneblers (3) einzurichten, um die Abgabe des Medikament-Aerosols an einen derartigen Patienten zu optimieren.

2. System (1) nach Anspruch 1, wobei das Atmungsmanöver Folgendes umfasst:
(i) Verändern eines normalen Tidalvolumens und einer normalen Strömungsrate, die der Gasströmung zugehörig sind, während eines ersten vorgegebenen Zeitraums,
(ii) Aktivieren einer Medikamentabgabevorrichtung, die konstruiert ist, um das Medikament zu vernebeln, während eines zweiten vorgegebenen Zeitraums, wobei der zweite vorgegebene Zeitraum kürzer dauert als der erste vorgegebene Zeitraum, und
(iii) Abgeben des vernebelten Medikaments an einen derartigen Patienten über die Gasströmung.

3. System (1) nach Anspruch 2, wobei das Verändern des normalen Tidalvolumens das Erhöhen des normalen Tidalvolumens umfasst.

4. System (1) nach Anspruch 2, wobei das Verändern der normalen Strömungsrate das Verringern der normalen Strömungsrate umfasst.

5. System (1) nach Anspruch 2, wobei das Verändern des normalen Tidalvolumens, das Verändern der normalen Strömungsrate und das Aktivieren der Medikamentabgabe im Wesentlichen gleichzeitig stattfinden.

6. System (1) nach Anspruch 1, wobei die Überwachungsvorrichtung (5) angeordnet ist, um drahtlos mit der Anzahl von Sensoren zu kommunizieren.

7. System (1) nach Anspruch 1, wobei die Überwachungsvorrichtung (5) angeordnet ist, um Signale zu empfangen, die mindestens einem von Folgendem zugehörig sind: Lungencompliance, Atemwegswiderstand, Kreislaufdruck, Atemfrequenz, Minutenvolumen, partieller Sauerstoffdruck im arteriellen Blut, partieller Kohlendioxiddruck im arteriellen Blut und Prozentsatz der Sauerstoffsättigung im arteriellen Blut.

8. System (1) nach Anspruch 1, wobei das System (1) ferner angeordnet ist, um vor der Abgabe des Atmungsmanövers und des optimierten Atmungsmanövers eine Angabe für einen Patienten zu erzeugen.

9. System (1) nach Anspruch 1, wobei das System (!) ferner angeordnet ist, um den Vernebler (3) vor einem Ende einer Einatmungsphase zu deaktivieren, um eine Periode ,sauberer Luft' bereitzustellen.

10. System (1) nach Anspruch 1, wobei das System (1) ferner angeordnet ist, um eine weitere Angabe zu erzeugen, um einen Patienten zu informieren, dass das Atmungsmanöver geendet hat.

11. System (1) nach Anspruch 8 oder 10, wobei die Angabe und/oder die weitere Angabe ein hörbares Signal, ein visuelles Signal und/oder ein taktiles Signal sind.

12. System (1) nach Anspruch 1, wobei das System (1) angeordnet ist, um eine Zeitdauer zwischen der Abgabe des Atmungsmanövers und eines nachfolgenden Atmungsmanövers in Abhängigkeit von der Reaktion des Patienten auf das Atmungsmanöver anzupassen.

13. System (1) nach Anspruch 12, wobei die Reaktion des Patienten auf das Atmungsmanöver die Herzfrequenz des Patienten einschließt.

## Revendications

1. Système (1) pour délivrer un médicament administré par aérosol à un patient ventilé, comprenant :
un ventilateur (2) structuré pour produire un flux de gaz respiratoire ;
un nébuliseur (3) structuré pour administrer un médicament par aérosol ;
un dispositif de contrôle (5) structuré pour produire un signal de sortie ; et
une unité de commande (6) couplée de manière opérationnelle au ventilateur (2), au nébuliseur (3) et au dispositif de contrôle (5), dans lequel l'unité de commande (6) est conçue pour :
coordonner le fonctionnement du ventilateur (2) et le nébuliseur (3) pour produire une manoeuvre respiratoire ;
**caractérisé en ce que** :
le système (1) comprend en outre un certain nombre de capteurs agencés pour être en communication avec le dispositif de contrôle (5), les capteurs étant agencés pour être appliqués au patient (4) pour suivre l'état physiologique du patient ;
dans lequel le signal de sortie est associé à une réponse d'un tel patient à la réception du médicament administré par aérosol ; et
dans lequel l'unité de commande (6) est en outre conçue pour, en réponse au signal de sortie provenant du dispositif de contrôle (5), établir automatiquement des paramètres de ventilateur (2) et des paramètres de nébuliseur (3) pour optimiser la délivrance du médicament administré par aérosol à un tel patient.

2. Système (1) selon la revendication 1, dans lequel la manoeuvre respiratoire comprend :
(i) la modification d'un volume respiratoire normal et d'un débit normal associé à un flux de gaz pendant une première période prédéterminée,
(ii) l'activation d'un dispositif de délivrance de médicament structuré pour administrer le médicament par aérosol pendant une seconde période prédéterminée, dans laquelle la seconde période prédéterminée a une durée qui est inférieure à une durée de la première période prédéterminée, et
(iii) la délivrance du médicament administré par aérosol à un tel patient via le flux de gaz.

3. Système (1) selon la revendication 2, dans lequel la modification du volume respiratoire normal comprend l'augmentation du volume respiratoire normal.

4. Système (1) selon la revendication 2, dans lequel la modification du débit normal comprend la diminution du débit normal.

5. Système (1) selon la revendication 2, dans lequel la modification du volume respiratoire normal, la modification du débit normal et l'activation du dispositif de délivrance de médicament se produisent sensiblement simultanément.

6. Système (1) selon la revendication 1, dans lequel le dispositif de contrôle (5) est agencé pour communiquer sans fil avec le nombre de capteurs.

7. Système (1) selon la revendication 1, dans lequel le dispositif de contrôle (5) est agencé pour recevoir des signaux associés à au moins l'un de l'élasticité pulmonaire, de la résistance des voies aériennes, de la pression de circuit, du débit respiratoire, du volume minute, de la pression partielle d'oxygène dans le sang artériel, de la pression partielle de dioxyde de carbone dans le sang artériel, et du pourcentage de saturation d'oxygène dans le sang artériel.

8. Système (1) selon la revendication 1, dans lequel le système (1) est en outre agencé pour produire une indication à un patient avant la délivrance de la manoeuvre respiratoire et de la manoeuvre respiratoire optimisée.

9. Système (1) selon la revendication 1, dans lequel le système (1) est en outre agencé pour désactiver le nébuliseur (3) avant une fin d'une phase d'inhalation pour fournir une période « d'air propre ».

10. Système (1) selon la revendication 1, dans lequel le système (1) est en outre agencé pour produire une indication supplémentaire pour informer un patient que la manoeuvre respiratoire a fini.

11. Système (1) selon la revendication 8 ou 10, dans lequel l'indication et/ou l'indication supplémentaire est un signal audible, un signal visuel, et/ou un signal tactile.

12. Système (1) selon la revendication 1, dans lequel le système (1) est agencé pour ajuster une période de temps entre la délivrance de la manoeuvre respiratoire et une manoeuvre respiratoire suivante en fonction de la réponse du patient à la manoeuvre respiratoire.

13. Système (1) selon la revendication 12, dans lequel la réponse du patient à la manoeuvre respiratoire inclut le rythme cardiaque du patient.
